# EUROPÄISCHE PATENTSCHRIFT

(11) **EP 1 357 817 B1**
(45) Veröffentlichungstag und Bekanntmachung des Hinweises auf die Patenterteilung: **21.03.2007**
(21) Anmeldenummer: 02701269.9
(22) Anmeldetag: 06.02.2002
(51) Int. Cl.: A45D 20/10, A45D 20/42, A47K 10/48

(54) **WARMLUFTGERÄT**
HOT AIR APPLIANCE
APPAREIL A AIR CHAUD

(30) Priorität: 10.02.2001 DE 10106109
(43) Veröffentlichungstag der Anmeldung: 05.11.2003
(73) Patentinhaber: Wella Aktiengesellschaft, 64274 Darmstadt (DE)
(72) Erfinder: ALLWOHN, Jürgen, 65558 Burgschwalbach (DE); MATTINGER, Detlef, 64404 Bickenbach (DE); UHL, Stefan, 64319 Pfungstadt (DE)
(86) Internationale Anmeldenummer: PCT/EP2002/001210
(87) Internationale Veröffentlichungsnummer: WO 2002/063991

(56) Entgegenhaltungen:
- EP-A- 0 176 003
- DE-A- 2 363 820
- DE-A- 19 915 377

## Beschreibung

Die Erfindung betrifft einen Handhaartrockner nach der Gattung des Oberbegriffs des Anspruchs 1.

Handhaartrockner mit Heizung und Gebläse zur Trocknung oder Wärmebehandlung von Kopfhaar sind in den verschiedensten Ausführungen bekannt, z.B. als sogenannter Fön (Handhaartrockner zum Trocknen und Stylen von Haar) . Geräte dieser Art arbeiten im Allgemeinen zuverlässig und erfüllen ihren Bestimmungszweck.

Gattungsfremde Warmluftgeräte sind aus der JP10248512A als ein elektrisches Raumheizgerät und aus der JP03241264A als ein elektrisches Fußwärmgerät bekannt, die mit einem katalytischen Geruchsfilter versehen sind zur Verbesserung der Raumluft.

Der Erfindung liegt demgegenüber die Aufgabe zu Grunde, einen Handhaartrockner nach der Gattung des Oberbegriffs zu schaffen, der durch einfache Maßnahmen eine weitere Funktion bzw. einen weiteren Bestimmungszweck erfüllt.

Diese Aufgabe wird nach den kennzeichnenden Merkmalen des Anspruchs 1 gelöst. Weitere vorteilhafte Weiterbildungen gehen aus den Unteransprüchen hervor.

In einem Friseursalon herrscht eine oft unangenehme Mischung unterschiedlichster Geruchsstoffe vor, typischerweise Ammoniak und Parfüme. Zusätzlich zur Geruchsbelästigung für Kunden und Friseure besteht durch die oben genannten Stoffe vor allem für das Friseurpersonal ein gewisses Allergierisiko. Eine effektive Reinigung der Raumluft im Salon erhöht den Kundenkomfort und verbessert spürbar die Arbeitsbedingungen. Zu diesem Zweck werden dem Friseur verschiedene Raumluftreiniger angeboten. Nachteil dieser Geräte ist, dass sie zusätzlich Geld kosten, dass sie Energie verbrauchen, und dass sie sowohl Platz als auch eine eigene Stromversorgung benötigen.

Die Lösung der oben genannten Nachteile erfolgt durch eine Integration eines gattungsgleichen Handhaartrockners für beispielweise eine Kopfhaarbehandlung. Insbesondere bei Handhaartrocknern entstehen durch Mehrfachnutzung Synergien, die diesen Zusatznutzen besonders ökonomisch und rationell gestalten. Dadurch entfällt auch eine Notwendigkeit eines separaten Luftreinigers. Auch ergeben sich dadurch neue Gerätegenerationen mit einer neuen zusätzlichen Funktionalität, wobei beispielsweise der Friseur bei seiner täglichen Arbeit mit dem Handhaartrockner automatisch die Luft im Salon reinigt.

Gegenstand der Erfindung ist ein Handhaartrockner mit integriertem, katalytischem Geruchsfilter, wobei wahlweise die Gerätekomponenten Gebläse/Heizung oder auch nur das Gebläse zusätzlich genutzt bzw. mit einer luftreinigenden Funktionalität ausgestattet sind. Katalysatoren, die Geruchsstoffe in der Luft zersetzen sollen, benötigen eine hohe Arbeitstemperatur und im Sinne einer Effizienz einen hohen Luftdurchsatz. Beide Bedingungen sind in einem Handhaartrockner bzw. Fön bereits in idealer Weise gegeben. Die Arbeitstemperatur des Katalysators wird durch thermische Kopplung mit dem Heizelement gewährleistet. Vorzugsweise ist der Heizdraht direkt mit der katalytisch wirksamen Substanz beschichtet. Es ist aber auch denkbar, dass ein separates Katalysatorfilterelement in ausreichender Nähe zum Heizkörper angebracht ist um die Betriebstemperatur sicherzustellen. Es ist ebenfalls möglich, dieses Element mit einer eigenen Heizung auszustatten, wenn sich dadurch konstruktive Vorteile ergeben. In diesem Fall wird die entstehende Abwärme zusätzlich zum Erhitzen des Luftstromes genutzt. Der benötigte Luftdurchsatz ist automatisch gewährleistet, da das Filterelement direkt in den Luftströmungsweg des Gerätes integriert wird. Die Kombination eines derartigen Filters mit einem friseurtechnischen Handhaartrockner bietet dem Friseur eine preiswerte und praktische Möglichkeit, die Luft in seinem Salon zu verbessern.

Die Erfindung wird an Hand von drei verschiedenen, schematisch dargestellten Ausführungsbeispielen näher beschrieben.

Es zeigt:
- Fig.1: ein erstes Ausführungsbeispiel eines Handhaartrockners mit einem katalytischen Geruchsfilter;
- Fig. 2: eine Schnittdarstellung eines vergrößert dargestellten einzelnen Heizdrahts mit einer Katalysatorsubstanzbeschichtung;
- Fig. 3: ein zweites Ausführungsbeispiel eines Handhaartrockners mit einem katalytischen Geruchsfilter, und
- Fig. 4: ein drittes Ausführungsbeispiel eines Handhaartrockners als Wärmestrahler mit Gebläse und einem katalytischen Geruchsfilter.

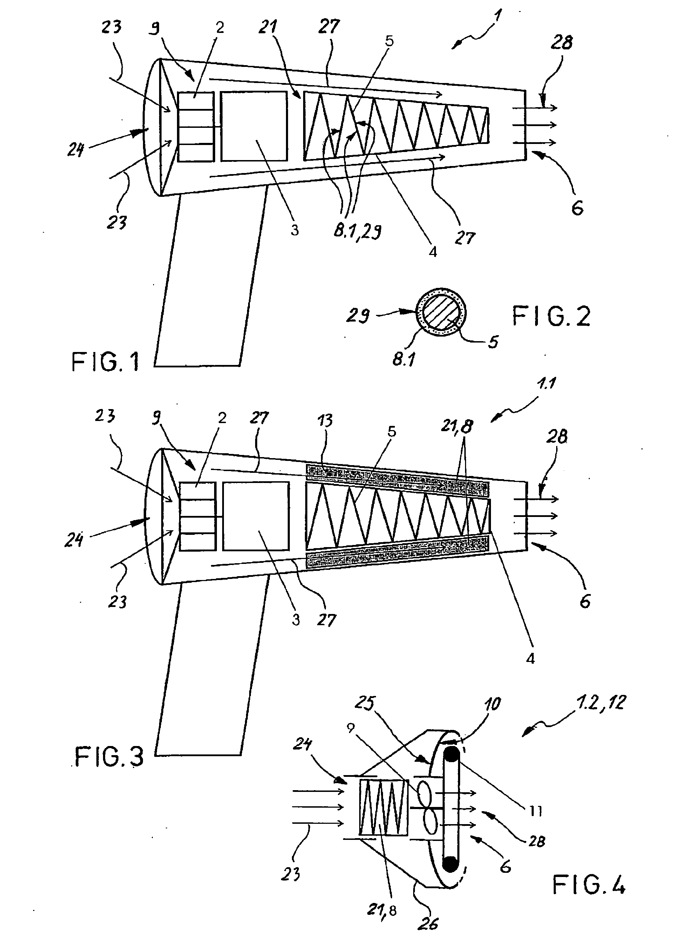

Fig. 1 zeigt einen Handhaartrockner 1 mit einem integrierten katalytischen Geruchsfilter 21 anhand eines prinzipiell dargestellten typischen Handhaartrockners 1 als ein erstes Ausführungsbeispiel. Bei dem Handhaartrockner 1 wird Außenluft 23 durch ein von einem Motor 3 angetriebenes Gebläserad 2 über einen Lufteinlass 24 angesaugt, wobei ein Gebläseluftstrom 27 über den Heizkörper 4 geführt und durch den Luftauslass 6 als ein Warmluftstrom 28 ausgeblasen wird. Ein solcher Handhaartrockner 22 weist bereits alle Grundmerkmale auf, die für einen katalytischen Geruchsfilter 21 benötigt werden: Es besteht ein hoher Luftdurchsatz und an mindestens einer Stelle im Bereich einer Luftführung eine deutlich erhöhte Temperatur. Der Luftdurchsatz wird benötigt, um die Raumluft möglichst gründlich zu reinigen sowie die hohe Temperatur, um die Funktion eines Katalysators (katalytischer Luftfilter 21) sicherzustellen. Um die gewünschten katalytischen Reaktionen effizient zu gestalten, sollte die katalytisch wirksame Oberfläche 29 thermisch möglichst gut mit dem Heizkörper 4 gekoppelt sein. Dies wird vorzugsweise dadurch erreicht, dass der Heizkörper 4 selbst oder nur Teile davon, wie beispielsweise der Heizdraht 5 direkt mit einer Katalysatorsubstanzbeschichtung 8.1 versehen ist oder zumindest teilweise aus einer solchen bestehen (Fig. 2), wobei der Geruchsfilter 21 mit der Katalysatorsubstanzbeschichtung 8.1 von dem Gebläseluftstrom 27 beaufschlagt wird. Eine solche Katalysatorsubstanzbeschichtung 8.1 ist beispielsweise aus der DE19915377A1 (die hiermit als vollständig geoffenbart gilt) bekannt als eine katalytische Zusammensetzung für Desodorisierungs- oder Oxidationszwecke, die eine Beschichtung aus einer Beschichtungsmasse auf einem Träger umfasst und erhältlich ist durch Aufbringen der Beschichtungsmasse, umfassend ein Polykondensat aus
(A) einem oder mehreren Silanen der allgemeinen Formel

   Rₐ-Si-X₍₄₋ₐ₎,

   worin die Reste R gleich oder verschieden sind und nicht hydrolysierbare Gruppen darstellen, die Reste X gleich oder verschieden sind und hydrolysierbare Gruppen oder Hydroxylgruppen bedeuten und a den Wert 0,1, 2 oder 3 hat, wobei bei mindestens 50 Stoffmengen-% der Silane a größer 0 ist, oder einem davon abgeleiteten Oligomer,
(B) gegebenenfalls einer oder mehreren Verbindungen von glasbildenden Elementen, und Teilchen von einem oder mehreren Übergangsmetalloxiden, wobei das Gewichtsverhältnis von Übergangsmetalloxid-Teilchen zu Polykondensat 10:1 bis 1:10 beträgt, auf den Träger und thermisches Behandeln der aufgebrachten Beschichtungsmasse.
Es ist außerdem möglich und sinnvoll, sämtliche luftführenden Bereiche eines Handhaartrockners 1,1.1,1.2 mit katalytisch wirksamen Oberflächen 29 zu versehen, sofern sie im Betrieb eine zur Aktivierung des Katalysators 8 ausreichende Temperatur erreichen.

In einem zweiten Ausführungsbeispiel nach der Fig. 3 ist ein separater Katalysator 8 mit einer eigenständigen Struktur in den Luftweg eines Handhaartrockners 1.1 integriert, wodurch diese Struktur so gewählt ist, dass eine große Oberfläche vorliegt und diese gut vom Gebläseluftstrom 27 umspült wird. Dies kann zum einen hinsichtlich einer Maximierung einer aktiven Fläche sinnvoll sein. Zum anderen ist es bei Heizkörpern 4 sinnvoll, die nicht primär zur Lufterwärmung genutzt werden, sondern zum Beispiel nach der Fig.4 als Wärmestrahler 12 mit einem Quarzstrahler 11 ähnlich wie beispielsweise nach der EP0695552B1 konzipiert sind. Dabei kann das separate Katalysatorelement 8 entweder thermisch passiv mit dem Heizkörper 4 gekoppelt sein oder selbst aktiv mittels eines separaten Wärmestrahlers 13 beheizt und vom Luftstrom des Gebläses 9 beaufschlagt werden.

In einem dritten Ausführungsbeispiel eines Handhaartrockners 1.2 nach der Fig. 4 saugt ein Gebläse 9 Außenluft 23 über ein separates Katalysatorelement 8 an. Der hier ringartig ausgestaltete Quarzstrahler 11 in einem Gehäuse 26 ist nicht primär zur Lufterwärmung ausgelegt. Diese erfolgt hauptsächlich durch die Abwärme an der Rückseite 25 eines Reflektors 10. Wenn der Katalysator 8 bzw. Geruchsfilter 21 zusätzlich beheizt wird, um die nötige Betriebstemperatur zu erreichen, trägt dies zur ohnehin erwünschten Erwärmung der Luft bei. Der Geruchsfilter 21 nach den Fig. 3 und 4 ist derart ausgebildet, dass dieser zwecks Reinigung entnehmbar ist.

Weitere Katalysatormaterialien bzw. Katalysatorbeschichtungen 8.1 oder bevorzugte Bauformen von Katalysatorelementen 8 werden an dieser Stelle nicht näher beschrieben, da sie aus dem Stand der Technik - wie beispielsweise aus der DE19915377A1 - hinreichend vorbekannt sind. Besonders geeignet sind jedoch Materialien, die einerseits die in einem Friseursalon vorkommende Geruchsstoffe wie beispielsweise Ammoniak, Mercaptane und Parfüme möglichst gut und bei möglichst geringen Temperaturen abbauen und sich andererseits einfach und kostengünstig auf typische Heizelemente und/oder andere Oberflächen in der Luftführung eines Handhaartrockners 1 aufbringen lassen. Sie sollten eine gute und dauerhafte Haftung aufweisen und Leistung und Lebensdauer der Heizkörper 4 nicht beeinträchtigen.

### Bezugsziffernliste:

- 1,1.1,1.2: Handhaartrockner
- 2: Gebläserad
- 3: Motor
- 4: Heizkörper
- 5: Heizdraht
- 6: Luftauslass
- 8: Katalysatorelement
- 8.1: Katalysatorsubstanzbeschichtung
- 9: Gebläse
- 10: Reflektor
- 11: Quarzstrahler
- 12: Wärmestrahler
- 13: Wärmestrahler
- 21: Geruchsfilter
- 23: Außenluft
- 24: Lufteinlass
- 25: Rückseite
- 26: Gehäuse
- 27: Gebläseluftstrom
- 28: Warmluftstrom
- 29: Oberfläche

## Patentansprüche

1. Handhaartrockner mit einem elektrischen Heizkörper und einem Gebläse zur Erzeugung eines Warmluftstromes, **dadurch gekennzeichnet, dass** im Handhaartrockner (1,1.1,1.2) ein katalytischer Geruchsfilter (21) integriert ist, der von einem Gebläseluftstrom (27) beaufschlagt ist.

2. Handhaartrockner nach Anspruch 1, **dadurch gekennzeichnet, dass** der Geruchsfilter (21) im luftführenden Bereich des Gebläses (9) angeordnet und mit katalytisch wirksamen Oberflächen (29) versehen ist, wobei die Oberfächen (29) vom Heizkörper (4) aus eine ausreichende Betriebstemperatur zur Aktivierung des Katalysators (8) erhalten.

3. Handhaartrockner nach Anspruch 1, **dadurch gekennzeichnet, dass** der katalytische Geruchsfilter (21) als ein separates Katalysatorelement (8) vorgesehen ist, welches von der Wärme eines separaten Wärmestrahlers (13) und vom Luftstrom des Gebläses (9) beaufschlagt ist.

4. Handhaartrockner nach Anspruch 1, **dadurch gekennzeichnet, dass** der katalytische Geruchsfilter (21) durch eine Katalysatorsubstanzbeschichtung (8.1) eines Heizdrahts (5) des Heizkörpers (4) gebildet ist.

5. Handhaartrockner nach mindestens Anspruch 3, **dadurch gekennzeichnet, dass** der Geruchsfilter (21) aus dem Handhaartrockner (9,1.1,1.2) zwecks Reinigung entnehmbar ausgebildet ist.

## Claims

1. Handheld hair-dryer having an electric heater and a blower for generating a hot-air stream, **characterized in that** a catalytic odor filter (21), which is acted on by a blower air stream (27), is integrated in the handheld hair-dryer (1, 1.1, 1.2).

2. Handheld hair-dryer according to Claim 1, **characterized in that** the odor filter (21) is arranged in the air-carrying region of the blower (9) and is provided with catalytically active surfaces (29), the surfaces (29), from the heater (4), acquiring an operating temperature which is sufficient to activate the catalyst (8).

3. Handheld hair-dryer according to Claim 1, **characterized in that** the catalytic odor filter (21) is provided as a separate catalyst element (8) which is acted on by the heat of a separate thermal radiator (13) and by the air stream from the blower (9).

4. Handheld hair-dryer according to Claim 1, **characterized in that** the catalytic odor filter (21) is formed by a coating of catalytic substance (8.1) on a heater wire (5) of the heater (4)..

5. Handheld hair-dryer according to at least Claim 3, **characterized in that** the odor filter (21) is designed so that it can be removed from the handheld hair-dryer (1, 1.1, 1.2) in order to be cleaned.

## Revendications

1. Sèche-cheveux à main comprenant un corps chauffant électrique et une soufflante pour produire un courant d'air chaud, **caractérisé en ce que** l'on intègre dans le sèche-cheveux à main (1, 1.1, 1.2) un filtre à odeurs catalytique (21) qui est sollicité par un courant d'air de la soufflante (27).

2. Sèche-cheveux à main selon la revendication 1, **caractérisé en ce que** le filtre à odeurs (21) est disposé dans la région de la soufflante (9) guidant l'air et est pourvu de surfaces (29) à action catalytique, les surfaces (29) obtenant du corps chauffant (4) une température de fonctionnement suffisante pour activer le catalyseur (8).

3. Sèche-cheveux à main selon la revendication 1, **caractérisé en ce que** le filtre à odeurs catalytique (21) est prévu sous la forme d'un élément de catalyseur séparé (8) qui est sollicité par la chaleur d'un dispositif de chauffage par rayonnement séparé (13) et par le courant d'air de la soufflante (9).

4. Sèche-cheveux à main selon la revendication 1, **caractérisé en ce que** le filtre à odeurs catalytique (21) est formé par un revêtement de substance de catalyseur (8.1) d'un fil métallique chauffant (5) du corps chauffant (4).

5. Sèche-cheveux à main selon au moins la revendication 3, **caractérisé en ce que** le filtre à odeurs (21) est réalisé de manière à pouvoir être enlevé du sèche-cheveux à main (1, 1.1, 1.2) à des fins de nettoyage.
